(19) Europäisches Patentamt
European Patent Office
Office européen des brevets

(11) Veröffentlichungsnummer: **0 238 002**
**B1**

# (12) EUROPÄISCHE PATENTSCHRIFT

(45) Veröffentlichungstag der Patentschrift: **19.07.89**

(51) Int. Cl.⁴: **C07C 69/533,** C07C 67/00

(21) Anmeldenummer: **87103751.1**

(22) Anmeldetag: **14.03.87**

## (54) Verfahren zur Herstellung von Alkencarbonsäureestern.

(30) Priorität: **19.03.86 DE 3609138**

(43) Veröffentlichungstag der Anmeldung: **23.09.87 Patentblatt 87/39**

(45) Bekanntmachung des Hinweises auf die Patenterteilung: **19.07.89 Patentblatt 89/29**

(84) Benannte Vertragsstaaten: **BE CH DE FR GB IT LI NL**

(56) Entgegenhaltungen:
**US-A- 3 182 077**

**CHEMICAL ABSTRACTS, Band 82, Nr. 19, 12. Mai 1975, Columbus, Ohio, USA Y.MURAMOTO et al. "Alcoholysis of lactones in the presence of magnesium alkoxide. Preparation of 6-hydroxyhexanoates and 5-hydroxypentanoates" Seiten 505,506, Spalten 2,1, Zusammenfassung Nr. 124 673j 000**

(73) Patentinhaber: **BASF Aktiengesellschaft, Carl-Bosch-Strasse 38, D-6700 Ludwigshafen(DE)**

(72) Erfinder: **Kummer, Rudolf, Dr., Kreuzstrasse 6, D-6710 Frankenthal(DE)**
Erfinder: **Vagt, Uwe, Dr., Paul-Neumann-Strasse 44, D-6720 Speyer(DE)**
Erfinder: **Fischer, Rolf, Dr., Bergstrasse 98, D-6900 Heidelberg(DE)**
Erfinder: **Seufert, Walter, Dr., Laerchenweg 19, D-6720 Speyer(DE)**
Erfinder: **Becker, Rainer, Dr., Im Haseneck 22, D-6702 Bad Duerkheim(DE)**

**EP 0 238 002 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Aus Nippon Nogei Kogaku Kaishi 1974, 48, Seiten 525 bis 527; C.A. 82, 124.673 (1975) ist bekannt, daß man omega-Hydroxycarbonsäureester durch Umsetzen von 5- bis 7-gliedrigen Lactonen mit Alkoholen in Gegenwart von Magnesiumalkoholaten oder Säuren erhält.

Es war die technische Aufgabe gestellt, ein einstufiges Verfahren zur Herstellung von Alkencarbonsäureestern mit endständiger Doppelbindung, ausgehend von entsprechenden 6- und 7-gliedrigen Lactonen zur Verfügung zu stellen.

Diese Aufgabe wird gelöst in einem Verfahren zur Herstellung von Alkencarbonsäureestern der Formel

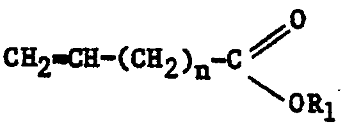

I ,

in der $R_1$ einen Alkylrest mit 1 - 6 Kohlenstoffatomen bezeichnet und n für 2, 3 oder 4 steht durch Umsetzen von Lactonen der Formel II

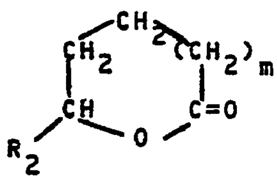

II ,

in der $R_2$ ein Wasserstoffatom oder eine Methylgruppe bezeichnet und m für 1 oder 2 steht mit Alkanolen mit 1 bis 6 Kohlenstoffatomen in Gegenwart von sauren Katalysatoren, dadurch gekennzeichnet, daß man die Umsetzung bei einer Temperatur von 150 bis 400°C durchführt.

Das neue Verfahren hat von Vorteil, daß man in einer Stufe, ausgehend von 6- und 7-gliedrigen Lactonen in guten Ausbeuten zu Alkencarbonsäureestern mit endständiger Doppelbindung gelangt. Ferner hat das neue Verfahren den Vorteil, daß es technisch auf einfache Weise durchführbar ist.

Das neue Verfahen ist insofern bemerkenswert, als die Lactonspaltung zu Alkencarbonsäureestern gegenüber der ebenfalls sauer katalysierten Etherbildung aus den beteiligten Alkoholen bevorzugt ist und trotz der mitverwendeten Katalysatoren die Doppelbindung endständig erhalten wird.

Erfindungsgemäß geht man von Lactonen der Formel II aus. Geeignete Lactone sind beispielsweise Caprolactan, 7-Methylcaprolacton, δ-Valerolacton oder 6-Methylvalerolacton. Bevorzugte Ausgangsstoffe sind Caprolacton, δ-Valerolacton und 6-Methylvalerolacton.

Die Umsetzung wird mit Alkanolen mit 1 bis 6 Kohlenstoffatomen durchgeführt. Geeignete Alkanole sind beispielsweise Methanol, Ethanol, Propanol, Butanol, Isopropanol oder Isobutanol, sekundäre Butanole n-Pentanol oder n-Hexanol. Besonders geeignet sind Methanol, Ethanol und Propanole.

Das Molverhältnis von Lactonen der Formel II zu Alkanolen beträgt vorteilhaft von 1 : 0,5 bis 1 : 10, insbesondere 1 :1 bis 1 : 5.

Die Umsetzung führt man bei einer Temperatur von 150 bis 400°C durch. Vorteilhaft hält man eine Temperatur von 200 bis 400°C, insbesondere 250 bis 400°C ein. In der Regel führt man die Umsetzung bei einem Druck von o,1 bis 100 bar, insbesondere 0,5 bis 10 bar durch.

Die Umsetzung wird in Gegenwart von sauren Katalysatoren durchegeführt. Geeignete Katalysatoren sind z.B. sauer wirkende Oxide von Elementen der dritten und vierten Hauptgruppe sowie der 4. bis 6. Nebengruppe des periodischen Systems, sowie Protonen- oder Lewisssäuren.

Die Umsetzung kann diskontinuierlich oder kontinuierlich als Festbettreaktion met Festbettkatalysatoren, beispielsweise in Sumpf- oder Rieselphase, in der Flüssigphase, Gasphase oder im Wirbelbett, ferner mit in der Flüssigphase suspendierten Festbettkatalysatoren durchgeführt werden.

Als saure Katalysatoren eignen sich beispielsweise hetereogene Katalysatoren wie Siliciumdioxid in Form von Kieselgel, Kieselgur, Quarz, weiterhin Titandioxid, Zirkondioxid, Phosphorpentoxid, Vanadiumpentoxid, Bortrioxid, Aluminiumoxid, Chromoxide, Molybdänoxide, Wolframoxide oder Gemische derselben.

Setzt man Lactone der Formel II an derartigen Festbettkatalysatoren in der Gasphase oder im Wirbelbett um, so hält man vorteilhaft eine Katalysatorbelastung von 0,1 bis 10, insbesondere von 1 bis 5 g Lacton der Formel II je g Katalysator und Stunde ein.

Es ist weiterhin möglich in der Flüssigphase homogen gelöste saure Katalysatoren zu verwenden. Geeignet sind beispielsweise Mineralsäuren, wie Schwefelsäure, Phosphorsäure, Salzsäure, Salpetersäure, Bromwasserstoff säure oder Sulfonsäuren wie Benzolsulfonsäure oder p-Toluolsulfonsäure. Das Molverhältnis von Lacton der Formel II zu Mineralsäure beträgt beispielsweise 1 : 0,001 bis 1 : 1, insbesondere 1 : 0,01 bis 1 : 0,1.

Die Umsetzung von Lactonen der Formel II mit Alkanolen in der Flüssigphase läßt sich beispielsweise so durchführen, daß man ein Gemisch aus Lactonen der Formel II und dem verwendeten Alkanol in Gegenwart eines suspendierten Festbettkatalysators, wie oben ausgeführt, oder eines homogen gelösten Katalysators auf die gewünschte Reaktionstemperatur erhitzt. Nach Ablauf der Reaktion, z.B. 0,5 bis 5 Stunden wird das Reaktionsgemisch abgekühlt und der saure Katalysator, z.B. durch Filtration oder Neutralisation entfernt. Das Reaktionsgemisch wird zweckmäßig anschließend zur Gewinnung der gewünschten Alkencarbonsäureester fraktioniert destilliert.

Eine bevorzugte Ausführungsform des erfindungsgemäßen Verfahrens in der Gasphase oder in der Wirbelphase, sieht z.B. so aus, daß man ein Gemisch aus Lacton der Formel II und dem jeweiligen Alkanol zunächst verdampft und dann ggf. zusammen mit einem Inertgas, wie Stickstoff, Kohlendioxid oder Argon, bei der gewünschten Reaktionstemperatur gasförmig über einen fest angeordneten Katalysator oder in auf- und abwirbelnder Bewegung befindlichen Katalysator leitet. Besonders bewährt haben sich hierfür Siliciumdioxid, Aluminiumoxid, Titandioxid, Bortrioxid oder deren Gemische. Der Reaktionsaustrag wird nach Kondensation fraktioniert destilliert. Nicht umgesetztes Lacton der Formel II wird zweckmäßig wieder in die Reaktion zurückgeführt.

Die nach dem erfindungsgemäßen Verfahren erhältlichen ω-Alkencarbonsäureester sind vielseitig verwendbare Zwischenprodukte. So eignen sie sich z.B. für die Herstellung von ω-Formylcarbonsäureestern durch Hydroformylierung. Dieses sind wichtige Ausgangsverbindungen, z.B. für die Herstellung von Aminocapronsäureestern und Caprolactam.

Das Verfahren nach der Erfindung sei an folgenden Beispielen veranschaulicht.

Beispiel 1

Pro Stunde wurde ein Gemisch aus 8,3 g Valerolacton und 2,7 g Methanol (Molverhältnis 1 : 1) in einen Verdampfer gepumpt und von dort zusammen mit 3 Litern/h Stickstoff bei 300°C über 5 g eines $Al_2O_3$-Katalysators geleitet. Die gasförmigen Reaktionsausträge wurden kondensiert und gewogen. Der nach 20 Stunden Reaktionszeit aufgefangene Austrag von 192 g wurde fraktionierend destilliert. Hierbei wurden 40 g 4-Pentensäuremethylester (21 % d. Th.), 4 g eines Gemisches aus 2- und 3-Pentenestern (2 % d.Th.) und 76 g unumgesetztes Valerolacton, das 18 % 5-Methylbutyrolacton enthielt ( Σ der Lactone: 46 % d. Th.) erhalten.

Beispiel 2

Pro Stunde wurden 10 ml einer 50 Gew.-% 6-Methylvalerolacton-Lösung in Methanol (Molverhältnis Methanol : Lacton = 3,6 : 1) verdampft und mit 3 l Stickstoff/h über 10 g Aluminiumoxid bei einer Reaktionstemperatur von 300° geleitet.

Der gasförmige Reaktionsaustrag wurde kondensiert und unter vermindertem Druck einer Kurzwegdestillation unterzogen. Nach 50 h Versuchzeit wurden so 330 g Rohprodukt isoliert, das anschließend fraktionierend destilliert wurde.

Auf diese Weise erhielt man 148 g (49 % d. Th.) Hexensäuremethylester (46% 5-, 46% 4-cis/trans- und 8 % 3/2-Hexenester) vom Siedepunkt 93°C-97°C/150 mbar und 33 g (12 % d. Th.) unumgesetztes 6-Methylvalerolacton.

Beispiel 3

Pro Stunde wurden 100 ml einer 50 Gew.-% Caprolacton-Lösung in Methanol (Molverhältnis Methanol : Lacton = 3,6 : 1) bei 235 bis 240°C verdampft und mit 30 l/h Stickstoff über 50 g Aluminiumoxid bei einer Reaktionstemperatur von 300° geleitet.

Der gasförmige Reaktionsaustrag wurde kondensiert und unter vermindertem Druck einer Kurzwegdestillation unterzogen. Nach 20 h Versuchzeit wurden so 876 g Rohprodukt isoliert, das anschließend fraktionierend destilliert wurde.

Auf diese Weise erhielt man 685 g (61 % d. Th.) Hexensäuremethylester (89 % 5-, 10 % 4-cis/trans- und 1 % 3/2-Hexenester) vom Siedepunkt 88°C - 91°C/150 mbar.

Beispiel 4

Pro Stunde wurden 200 ml einer 50-gew.-%igen Caprolactonlösung in Methanol (Molverhältnis Methanol : Lacton = 3,6 : 1) bei 300°C verdampft und mit 70 l Stickstoff über 200 g (325 ml) Aluminiumoxid-Wirbelkontakt (Korngröße 0,1 - 0,3 mm) (Reaktionstemperatur 300°C) geleitet.

Der gasförmige Reaktionsaustrag wurde über eine Versuchzeit von 8 Stunden Kühlfallen kondensiert und anschließend im Vakuum fraktionierend destilliert..

Auf diese Weise erhielt man 595 g (70 % d.Th.) Hexensäuremethylester (33 % 5-, 65 % 4-cis/trans- und 2 % 3/2-Hexenester) vom Siedepunkt 95 - 96°C/150 mbar.

**Patentansprüche**

1. Verfahren zur Herstellung von Alkencarbonsäureestern der Formel I

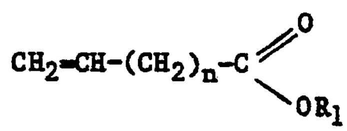

I ,

in der $R_1$ einen Alkylrest mit 1 bis 6 Kohlenstoffatomen bezeichnet und n für 2, 3 oder 4 steht, durch Umsetzen von Lactonen der Formel II

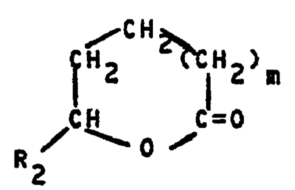

II ,

in der $R_2$ ein Wasserstoffatom oder eine Methylgruppe bezeichnet und m für 1 oder 2 steht mit Alkanolen mit 1 bis 6 Kohlenstoffatomen in Gegenwart von sauren Katalysatoren, dadurch gekennzeichnet, daß man die Umsetzung bei einer Temperatur von 150 bis 400°C durchführt.

2. Verfahren nach Anspruch 1, dadurch gekennzeichnet, daß man Caprolacton verwendet.

3. Verfahren nach Anspruch 1, dadurch gekennzeichnet, daß man δ-Valerolacton oder 6-Methylvalerolacton verwendet.

4. Verfahren nach den Ansprüchen 1 bis 3, dadurch gekennzeichnet, daß man als saure Katalysatoren Siliciumdioxid, Aluminiumoxid, Titandioxid, Bortrioxid oder deren Gemische verwendet.

5. Verfahren nach den Ansprüchen 1 bis 4, dadurch gekennzeichnet, daß man ein Molverhältnis von Lactonen der Formel II zu Alkanolen von 1 : 0,5 bis 1 : 10 einhält.

6. Verfahren nach den Ansprüchen 1 bis 5, dadurch gekennzeichnet, daß man die Umsetzung in der Gasphase durchführt.

7. Verfahren nach Anspruch 6, dadurch gekennzeichnet, daß man eine Katalysatorbelastung von 0,1 bis 10 g Lacton der Formel II je g Katalysator und Stunde einhält.

## Claims

1. A process for the preparation of an alkenecarboxylic ester of the formula I

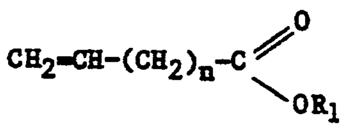

I

where $R_1$ is alkyl of 1 to 6 carbon atoms and n is 2, 3 or 4, by reacting a lactone of the formula II

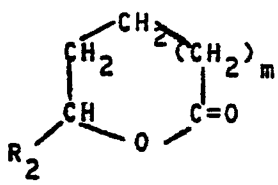

II

where $R_2$ is hydrogen or methyl and m is 1 or 2, with an alkanol of 1 to 6 carbon atoms in the presence of an acidic catalyst at from 150 to 400°C.

2. A process as claimed in claim 1, wherein caprolactone is used.

3. A process as claimed in claim 1, wherein δ-valerolactone or 6-methylvalerolactone is used.

4. A process as claimed in any of claims 1 to 3, wherein silicia, alumina, titanium dioxide, boron trioxide or a mixture thereof is used as the acidic catalyst.

5. A process as claimed in any of claims 1 to 4, wherein a molar ratio of lactone of the formula II to alkanol of from 1:10 is maintained.

6. A process as claimed in any of claims 1 to 5, wherein the reaction is carried out in the gas phase.

7. A process as claimed in claim 6, wherein a space velocity of from 0.1 to 10 g of lactone of the formula II per g of catalyst per hour is maintained.

## Revendications

1. Procédé de préparation d'esters d'acides alcènecarboxyliques de formule I

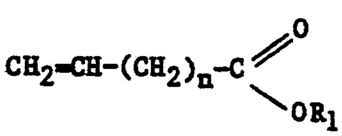

I ,

dans laquelle $R_1$ représente un reste alkyle ayant de 1 à 6 atomes de carbone et n est mis pour 2, 3 ou 4, par réaction de lactones de formule II

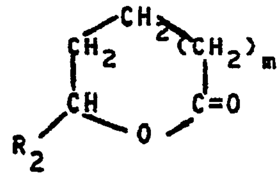

II ,

dans laquelle $R_2$ représente un atome d'hydrogène ou un groupement méthyle et m est mis pour 1 ou 2, avec des alcanols de 1 à 6 atomes de carbone en présence de catalyseurs acides, caractérisé en ce qu'on mène la réaction à une température de 150 à 400°C.

2. Procédé selon la revendication 1, caractérisé en ce qu'on utilise la caprolactone.

3. Procédé selon la revendication 1, caractérisé en ce qu'on utilise la δ-valérolactone ou la 6-méthylvalérolactone.

4. Procédé selon l'une des revendications 1 à 3, caractérisé en ce qu'on utilise comme catalyseur acide du dioxyde de silicium, de l'oxyde d'aluminium, du bioxyde de titane, du trioxyde de bore ou leurs mélanges.

5. Procédé selon l'une des revendications 1 à 4, caractérisé en ce qu'on maintient un rapport molaire des lactones de formule II aux alcanols de 1:0,5 à 1:10.

6. Procédé selon les revendications 1 à 5, caractérisé en ce qu'on mène la réaction en phase gazeuse.

7. Procédé selon la revendication 6, caractérisé en ce qu'on maintient une charge du catalyseur de 0,1 à 10g de lactone de formule II par g de catalyseur et par heure.